Europäisches Patentamt

European Patent Office

Office européen des brevets

(19)

(11) Veröffentlichungsnummer: **0 177 707**
**B1**

(12) # EUROPÄISCHE PATENTSCHRIFT

(45) Veröffentlichungstag der Patentschrift:
28.10.87

(21) Anmeldenummer: 85109977.0

(22) Anmeldetag: 08.08.85

(51) Int. Cl.⁴: **B 01 D 1/22,** F 17 C 13/02,
C 09 C 1/56

(54) Verfahren zur verbrauchsabhängigen Totalverdampfung von flüssigem Stickoxid.

(30) Priorität: 11.09.84 DE 3433301

(43) Veröffentlichungstag der Anmeldung:
16.04.86 Patentblatt 86/16

(45) Bekanntmachung des Hinweises auf die Patenterteilung:
28.10.87 Patentblatt 87/44

(84) Benannte Vertragsstaaten:
DE FR GB IT NL SE

(56) Entgegenhaltungen:
DE - A - 1 619 709
DE - A - 2 164 373
US - A - 2 456 889

(73) Patentinhaber: **Degussa Aktiengesellschaft,**
**Weissfrauenstrasse 9, D-6000 Frankfurt am Main 1 (DE)**

(72) Erfinder: **Engel, Richard, Dr., Veilchenweg 27,**
**D-5303 Bornheim-Waldorf (DE)**
Erfinder: **Voll, Manfred, Dr., Lindenstrasse 3,**
**D-6455 Erlensee (DE)**

## Beschreibung

Die Erfindung betrifft ein Verfahren zur verbrauchsabhängigen Totalverdampfung von flüssigem, überwiegend aus $N_2O_4$ bestehendem Stickoxid zur Bereitstellung dieses Gases für chemische Reaktionen, z.B. eine Oxidation der Oberfläche von Russen.

Bei der grosstechnischen Durchführung von Umsetzungen mit gasförmigen Stockoxiden $N_2O_4$ bzw. $NO_2$ ist es erforderlich, einen grösseren Mengenstrom dieses Reaktanden bereitzustellen und überlängere Zeit aufrechtzuerhalten. Während eine Entnahme kleinerer Mengenströme aus dem Gasraum der üblichen, mit flüssigem Stickoxid gefüllten Transport- oder Lagerbehälter keine Schwierigkeiten bereitet, reicht bei grösseren Mengenströmen die bei normalen Umgebungstemperaturen stattfindende Verdampfung nicht aus und müsste durch Heizung des Vorratsbehälters beschleunigt werden. Angesichts der Agressivität und Toxizität dieses Stoffes stellt jedoch eine Erwärmung eines grösseren Flüssigkeitsvolumens davon ein Sicherheitsrisiko dar.

Darüber hinaus tritt durch die bei der Verdampfung auftretende Abkühlung der Flüssigkeit eine Dampfdruckerniedrigung ein, was dazu führt, dass der Druck des verdampften Stickoxids umso weiter absinkt, je höher die Abnahmemenge ist. Bedingt durch den wechselnden Vordruck muss daher die Messung und Regelung der zu einer oder mehreren Verbrauchsstellen fliessenden Gasmenge laufend korrigiert werden, was wegen der Druck- und Temperatur-abhängigen Dissoziation des gasförmigen Distickstofftetroxids ohne aufwendige Rechner-gesteuerte Regelung kaum zu verwirklichen ist.

Daneben reagiert ein gespeichertes grösseres Volumen an flüssigem Stickoxid bei Wärmezufuhr mit einer gewissen Verzögerung, so dass bei wechselnden Abnahmemengen eine konstante Verdampfungstemperatur und damit ein konstanter Dampfdruck nicht trägheitslos einzustellen ist.

Weiterhin birgt die Beheizung eines grösseren Flüssigkeitsvolumens die Gefahr in sich, dass durch Siedeverzüge flüssiges Reagenz in die Dampfleitung gerät und damit zur Verbrauchsstelle durchschlägt, was angesichts der Reaktivität von Stickoxiden zu unkontrollierbaren Reaktionen führen kann.

Es bestand daher ein dringendes Bedürfnis, ein Verfahren zur praktisch trägheitslosen, verbrauchsabhängigen Totalverdampfung von flüssigem, überwiegend aus $N_2O_4$ bestehendem Stickoxid zu finden, das die erwähnten Schwierigkeiten überwindet.

Diese Aufgabe wird gemäss der Erfindung durch ein Verfahren gelöst, das dadurch gekennzeichnet ist, dass man das flüssige Stickoxid aus einer Vorlage über ein Puffergefäss dosiert einem Fallfilmverdampfer aufgibt, die nach Passieren von dessen Fallstrecke unverdampft gebliebene Restmenge Stickoxid in einem mit dem Ablauf des Fallrohres des Fallfilmverdampfers verbundenen Nachverdampfer verflüchtigt und den vereinigten Dampfstrom aus Fallfilmverdampfer und Nachverdampfer über mindestens eine beheizte, mit Flüssigkeitsdetektoren ausgerüstete Sicherheitsfalle der Verbrauchsstelle zuleitet, wobei die Flüssigkeitszufuhr zum Fallfilmverdampfer über den Druck im durchwegs beheizten Leitungssystem zwischen Fallfilmverdampfer und Verbrauchsstelle so geregelt wird, dass darin, unabhängig von der Abnahmemenge, ein konstanter vorgewählter Druck aufrechterhalten wird.

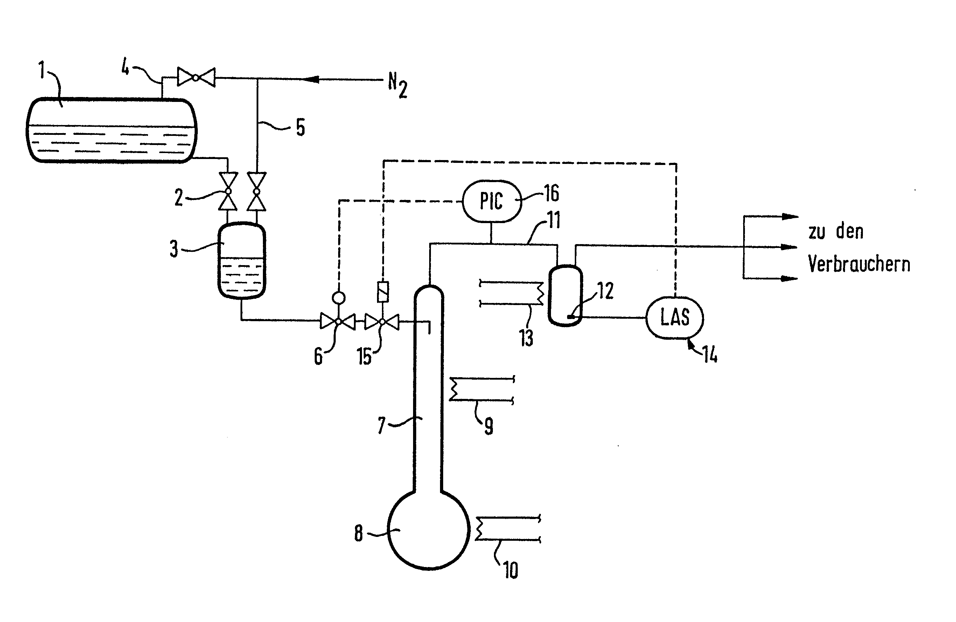

Das Verfahren wird im folgenden anhand einer Ausführungsform zur Stickoxid-Versorgung einer grösseren Anzahl von Anlagen zur oxidativen Nachbehandlung von Russen mit Stickoxid enthaltenden Gasen in Verbindung mit der Zeichnung näher erläutert.

Gemäss der einzigen Figur der Zeichnung befindet sich das flüssige Stickoxid in einer Vorlage 1, welches über ein Absperrorgan 2 mit dem Puffergefäss 3 in Verbindung steht. Auf die Vorlage und das Puffergefäss kann über die Leitungen 4 und 5 Stickstoff oder ein anderes Inertgas aufgedrückt werden, um den Gegendruck in der nachgeschalteten Verdampferanlage zu überwinden. Aus dem Puffergefäss gelangt das flüssige Stickoxid über ein Regelventil 6 auf den Verteilerkopf (nicht gezeigt) des Fallfilmverdampfers 7, der an seinem unteren Ende in ein als Nachverdampfer für eine im Fallfilmverdampfer unverdampft gebliebene Restmenge Stickoxid wirkendes Gefäss 8 mündet. Fallfilmverdampfer und Nachverdampfer sind über Heizungen 9 bzw. 10 auf Temperaturen oberhalb des bei einem gegebenen Gasdruck des Stickoxids vorliegenden Siedepunkts des letzteren erwärmbar. Das im Fallfilmverdampfer und Nachverdampfer verflüchtigte Stickoxid gelangt über die beheizte Leitung 11 und die durch Heizung 13 beheizte Sicherheitsfalle 12 über ebenfalls beheizte Leitungen zu den einzelnen Verbrauchern. Die Sicherheitsfalle ist im unteren Teil mit einem Flüssigkeitswächter 14 (z.B. einem auf den unterschiedlichen Brechungsindex von Flüssigkeit und Dampf ansprechenden optischen Gerät) ausgerüstet, der im Falle einer Störung durch in die Sicherheitsfalle überschlagendes flüssiges Stickoxid einen Alarm auslöst und/oder die Flüssigkeitszufuhr über das Schnellschlussventil 15 absperrt.

Ein konstanter, vorgewählter Druck vor den Verbraucherstellen wird erreicht, indem durch eine Druckmessung und Regelung (PIC) 16 das Regelventil 6 angesteuert wird.

Da das über das Regelventil 6 druckgeregelt zugeführte Stickoxid im Fallfilmverdampfer 7 und Nachverdampfer 8 totalverdampft wird, wird in der Leitung 11 bis zu den Verbrauchsstellen der vorgewählte Druck praktisch trägheitslos unabhängig von der Abnahmemenge konstant aufrechterhalten.

Alternativ kann anstelle des Regelventils 6 eine druckabhängig gesteuerte Dosierpumpe verwendet werden. Dabei kann unter Bedingungen, bei denen eine Verdampfung von Stickoxid auf der Saugseite der Pumpe ausgeschlossen ist, das Aufdrücken von Stickstoff über die Leitungen 4 bzw. 5 entfallen.

## Patentanspruch

Verfahren zur verbrauchsabhängigen Totalverdampfung von flüssigem, überwiegend aus N$_2$O$_4$ bestehendem Stickoxid, *dadurch gekennzeichnet,* dass man das flüssige Stickoxid aus einer Vorlage über ein Puffergefäss dosiert einem Fallfilmverdampfer aufgibt, die nach Passieren von dessen Fallstrecke unverdampft gebliebene Restmenge Stickoxid in einem mit dem Ablauf des Fallrohres des Fallfilmverdampfers verbundenen Nachverdampfer verflüchtigt und den vereinigten Dampfstrom aus Fallfilmverdampfer und Nachverdampfer über mindestens eine beheizte, mit Flüssigkeitsdetektoren ausgerüstete Sicherheitsfalle der Verbrauchsstelle zuleitet, wobei die Flüssigkeitszufuhr zum Fallfilmverdampfer über den Druck im durchwegs beheizten Leitungssystem zwischen Fallfilmverdampfer und Verbrauchsstelle so geregelt wird, dass darin, unabhängig von der Abnahmemenge, ein konstanter vorgewählter Druck aufrechterhalten wird.

## Claim

A process for the consumption-dependent total evaporation of liquid nitric oxide composed predominantly of N$_2$O$_4$, characterised in that the liquid nitric oxide is passed from a beaker, metered via a buffer vessel, to a falling film evaporator, the remainder of nitric oxide which is not evaporated after passing through the falling path thereof is volatilized in an after-evaporator connected to the outlet of the falling pipe of the falling film evaporator and the combined vapour stream from falling film evaporator and after-evaporator is fed through at least one heated safety valve equipped with liquid detectors to the place of use, wherein the liquid supply to the falling film evaporator is regulated by means of the pressure in the pipe system, which is heated throughout, between falling film evaporator and place of use in such a way that a constant predetermined pressure is maintained therein independently of the quantity reduced.

## Revendication

Procédé pour l'évaporation totale, en fonction de l'usage, d'oxydes d'azotes liquides, constitués principalement de N$_2$O$_4$, caractérisé en ce que l'on envoie d'un collecteur, en passant par un récipient tampon, l'oxyde d'azote liquide, en le dosant, dans un évaporateur à chute pelliculaire, la quantité résiduelle d'oxyde d'azote subsistant, sans avoir été évaporée, après ce parcours de chute, s'évaporant dans un post-évaporateur, relié avec la sortie du tube de chute de l'évaporateur à chute pelliculaire, les courants de vapeurs de l'évaporateur à chute pelliculaire et du post-évaporateur, réunis, étant envoyés, en passant par au moins une trappe de sécurité, chauffée, équipée de détecteurs de liquide, à l'endroit où ils seront utilisés, l'alimentation en liquide de l'évaporateur à chute pelliculaire étant réglée, au moyen de la pression régnant dans un système de conduites généralement chauffé, entre l'évaporateur à chute pelliculaire et le point d'utilisation, de façon telle que, indépendamment des quantités prélevées, il soit maintenu une pression prescrite constante.